# EUROPEAN PATENT APPLICATION

(11) **EP 2 878 412 A1**
(43) Date of publication of application: **03.06.2015**
(21) Application number: 13005518.9
(22) Date of filing: 27.11.2013
(51) Int. Cl.: B25G 3/18

(54) **Handgrip for an analyser**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Riwotzki, Karsten

(57) **Abstract**

In a first aspect, the present invention provides a handgrip (103) for an analyser (100), the analyser (100) having a groove into which an analysis strip (101) can be inserted. The handgrip (103) comprises a movable holding device (300) that can be moved between a proximal position and a distal position relative to the groove. The holding device (300) is adapted to retain (or hold) the analysis strip (101) when the holding device (300) is in the proximal position and to release the analysis strip (101) when the holding device (300) is in the distal position.

## Description

The present invention relates to a handgrip for an analyser having a groove into which an analysis strip can be inserted. The handgrip is aimed at extracting the analysis strip from the analyser after the strip has been inserted into the groove and processed by the analyser.

### BACKGROUND ART

Analysers based on inserting an analysis strip into a groove of the analyser are currently widely used. The strip is configured to receive and retain a body fluid (e.g. blood) sample to be analysed. The groove into which the strip is to be inserted to carry out an analysis (of e.g. glucose in blood) is typically arranged on an end region of the analyser. Once the strip has been used (so that it still contains a body fluid sample), the strip has to be removed from the analyser. Removal of the strip by hand is not recommended in order to avoid risk of infection, in particular when the analyser is used by or for a plurality of users, such as for a multitude of patients in a hospital environment.

Analysers are known which do not have any kind of system for ejecting strips. In this case, an operator (doctor, nurse, etc.) can remove a used strip from the analyser by gripping and pulling the strip by hand. But, as commented before, this way of extracting the strip is not recommended due to the reasons outlined above.

Analysers are known having a system for automatically ejecting strips integrated within the analyser, in such a way that all the components of the analyser (including the ejection system) are covered and/or protected by a common casing of the analyser. These ejection systems are typically based on rather complex configurations/mechanisms which are internal to the main casing of the analyser. Thus, if e.g. the ejection system needs to be repaired, the main casing of the analyser has to be dismounted, in which case other components, such as sensitive electronic elements, may be exposed to a certain risk of damage. Moreover, analysers (such as hand-held analysers for blood glucose measurement in a home environment or in a hospital environment) typically are protected against being manipulated by a user and, therefore, usually may not be opened in a non-destructive way.

Other known ejection systems integrated with an analyser are based on having a small wheel outside the main casing of the analyser and near the groove, the wheel being arranged to make a certain pressure on the strip (when inserted into the groove). In this case, the strip is ejected by an operator directly acting on (touching) the wheel to make it rotate, such that this rotation of the (pressing) wheel causes the strip to move out of the groove and, thus, to be ejected from the groove. A drawback of this approach may be that the operator has to act (by touching the wheel) on a position very close to the strip, so that the risk of contamination or infection may still be significant.

The present invention aims at providing an analysis strip ejection device for an analyser overcoming at least some of the problems/limitations of the prior art systems. In particular, the ejection device should allow hygienic handling of used analysis strips even with analysers which do not have an integrated analysis strip ejection mechanism. Moreover, the ejection device provided should be easy-to-use and should incur rather low costs.

### SUMMARY OF THE INVENTION

The above object(s) are solved by provision of a handgrip according to claim 1. Advantageous embodiments and additional features are contemplated in the dependent claims.

In a first aspect, the present invention provides a handgrip for an analyser, the analyser having a groove into which an analysis strip can be inserted. The handgrip comprises a movable holding device that can be moved between a proximal position and a distal position relative to the groove. The holding device is adapted to retain (or hold) the analysis strip when the holding device is in the proximal position and to release the analysis strip when the holding device is in the distal position.

The term "groove" as used herein comprises any configuration which may be suitable for interaction between an analyser, such as a hand-held analyser (e.g. for monitoring blood glucose and/or other analytes in a home environment or in a hospital environment), and an analysis strip, such as a test strip (for measurement of blood glucose and/or other analytes). Particularly, the term "groove" may encompass a slot, a cavity and/or an indentation adapted to receive an analysis strip.

This proposed configuration is based on "externally" gripping and pulling the strip away from the groove (by the holding device). This approach may provide a significant versatility in terms of e.g. providing different possibilities with respect to manufacture and/or use of the handgrip. For example, the handgrip could be fabricated as a removable handgrip, such that the handgrip may be removed from the analyser in a very easy manner. This way, a damaged handgrip may be easily replaced by a new or at least not damaged handgrip. Alternatively, the handgrip could be fabricated integrated with the analyser, in such a way that the analyser and the handgrip constitute a single product.

In any case, the handgrip, either a separate piece or a part of the analyser, may be repaired without necessity of dismounting the main casing of the analyser, such that other more sensitive components (such as e.g. electronic components) are not exposed to the risk of damage. Also, since the handgrip has a rather simple configuration and is external to the main casing of the analyser, the reparation of the handgrip may be easier and cheaper with respect to prior art ejection systems.

Moreover, this approach may permit a variety of configurations in which the holding device may be moved (between the proximal and distal positions) by a corresponding operator (e.g. doctor, nurse, etc.) acting on positions significantly away from the strip. This way, the risk of infection may be significantly minimized.

In some embodiments, the holding device may comprise a pressing arrangement adapted to retain the analysis strip by applying a pressure when the holding device is in the proximal position and to release the analysis strip by ceasing the pressure when the holding device is in the distal position. Alternatively, one or more elastic (instead of pressing) arrangements may be used to achieve the same or similar functionalities.

In particular embodiments, the pressing arrangement may comprise an upper pressing member and a lower pressing member adapted to apply the pressure to the analysis strip and cause the analysis strip to be stuck between the upper and lower pressing members.

In even more particular embodiments, the upper and lower pressing members may be coupled pivotally to each other, such that the upper and lower pressing members can pivot relative to each other for causing the analysis strip to swap between being stuck and not being stuck between the upper and lower pressing members, and vice versa.

Alternatively, the upper pressing member may be coupled pivotally to the lower pressing member, such that the upper pressing member can pivot relative to the lower pressing member for causing the analysis strip to swap between being stuck and not being stuck between the upper and lower pressing members, and vice versa.

Optionally, at least one of the upper and lower pressing members may have a recess such that the analysis strip is substantially press fitted into the recess when the analysis strip is stuck between the upper and lower pressing members. More particularly, each of the upper and lower pressing members may have a recess such that the analysis strip is substantially press fitted into both recesses when the analysis strip is stuck between the upper and lower pressing members. An aspect of these embodiments may be that the recess or recesses may constitute a kind of tunnel-shaped cavity with a height which may be significantly ensured and remained invariable (along the life of the handgrip) whenever the pressing arrangement is in the proximal position. This results in that the pressure on the strip may be kept optimally invariable (along the life of the handgrip) whenever the pressing arrangement is in the proximal position.

According to some embodiments, the handgrip may comprise a casing configured to receive the analyser in a press fitting manner, and the holding device may be movable relative to the casing.

In particular embodiments, the casing may comprise at least two flanges for pressing against corresponding end sides of the analyser to keep the analyser press fitted into the casing. In alternative embodiments, more than two flanges can be considered and some of them can be configured to press against other sides (e.g. lateral sides) of the analyser.

In some embodiments, the upper pressing member may comprise an upwards extension at its inner end. In the same or other embodiments, one of the flanges may be adapted to press against the end side of the analyser substantially having the groove, this flange comprising a top wall having a downwards extension at its outer end. The downwards extension of the top wall and the upwards extension of the upper pressing member may be arranged respective to each other in such a way that they engage during a part of the motion of the holding device between the proximal and distal positions, said engagement causing the abovementioned pivoting of the upper pressing member with respect to the lower pressing member.

According to embodiments of the handgrip, the casing may comprise a sheet-shaped portion connecting the at least two flanges, in such a way that a rear/bottom side of the analyser is at least partially covered by the sheet-shaped portion when the analyser is press-fitted into the casing.

In embodiments of the handgrip, the holding device may comprise a sheet-shaped portion slidably coupled to the sheet-shaped portion of the casing. This slidably coupling between the sheet-shaped portion of the holding device and the sheet-shaped portion of the casing may be based on e.g. a rail based mechanism. An aspect of these embodiments may be that an operator (e.g. doctor, nurse, etc.) may eject the strip by acting on (touching) the sheet-shaped portion of the holding device, which is significantly away from the strip/groove. The risk of infection is thus significantly minimized.

In still more particular embodiments, the sheet-shaped portion of the holding device may have an external rough surface. An aspect of this feature may be that the operator's hand (or fingers) may act on the sheet-shaped portion of the holding device in an improved manner.

Any of the previously commented embodiments of the handgrip may be made of plastic. More particularly, any of the embodiments may be made of Acrylonitrile butadiene styrene (ABS). This way, the handgrip may be significantly cheap to manufacture while providing a significant efficiency.

Preferably, in any of the previously described embodiments, the handgrip may be a removable handgrip, which may be based on e.g. the above mentioned handgrip casing configured to receive the analyser in a press fitting manner.

### BRIEF DESCRIPTION OF THE DRAWINGS

Particular embodiments of the present invention will be described in the following by way of non-limiting examples, with reference to the appended drawings, in which:
Figure 1 schematically represents a top view of an analyser press fitted into a handgrip according to an embodiment;
Figure 2 schematically represents a lateral view of an analyser press fitted into a handgrip similar to the one shown in Figure 1;
Figure 3 schematically represents a perspective view of a portion of a holding device suitable for a handgrip similar to the ones shown in previous Figures;
Figure 4 schematically represents a perspective view of a casing suitable for a handgrip similar to the ones shown in previous Figures;
Figure 5 schematically represents a perspective view of a holding device portion similar to the one shown in Figure 3, but turned upside down;
Figure 6 schematically represents a perspective view of a handgrip similar to the ones shown in previous Figures;
Figure 7 schematically represents a sectional lateral view of a handgrip similar to the ones shown in previous Figures;
Figure 8 schematically represents a sectional lateral view of a portion of a handgrip similar to the ones shown in previous Figures, with an analyser press fitted into the handgrip and a strip inserted into a pertinent groove of the analyser;
Figure 9 schematically represents a sectional lateral view of the handgrip portion of Figure 8, but with the strip being ejected; and
Figure 10 schematically represents a front view of an analyser press fitted into a handgrip similar to the ones shown in previous Figures, but from a particular point of view indicated in Figure 2.

### DETAILED DESCRIPTION OF EMBODIMENTS OF THE INVENTION

In the following description, numerous specific details are set forth in order to provide a thorough understanding of examples of the present invention. It will be understood by one skilled in the art however, that examples of the present invention may be practiced without some or all of these specific details. In other instances, well known elements have not been described in detail in order not to unnecessarily obscure the description of the present invention.

In some cases, the same reference numbers are used to indicate the same or similar elements shown in different Figures.

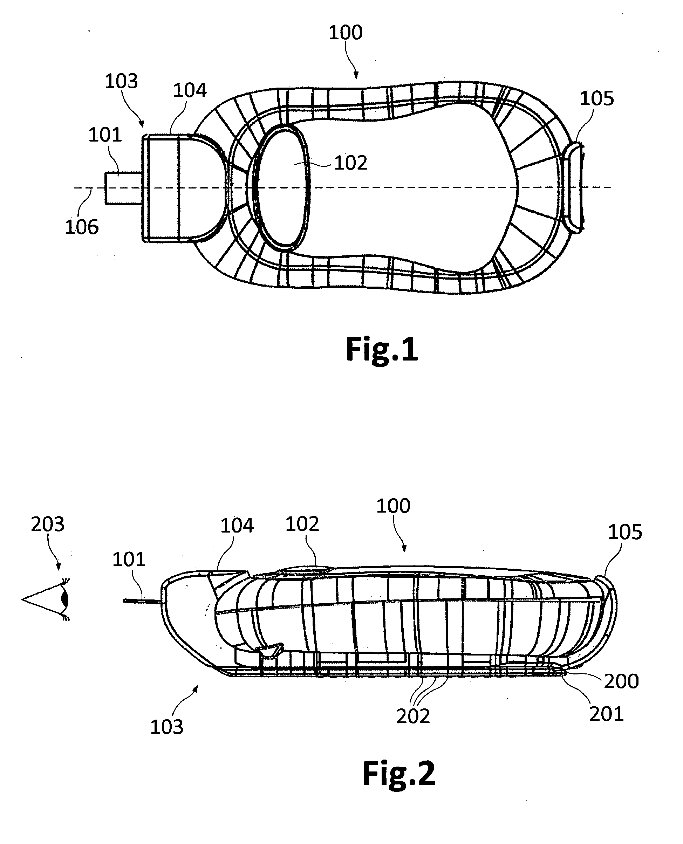

Figure 1 schematically represents a top view of an analyser 100 having a groove (not shown) into which an analysis strip 101 can be inserted, the analyser 100 being press fitted into a handgrip 103 according to an embodiment provided by the invention. The handgrip 103 comprises a movable holding device (not shown) that can be moved between a proximal (or receded) position and a distal (or extended) position relative to the groove. The analysis strip 101 is retained (or held) by the holding device when the holding device is in the proximal position (as shown in the Figure) and is released when the holding device is in the distal position (not shown in this Figure). Details about the holding device and its movement between proximal (retaining) and distal (realising) positions are provided in other parts of the description with reference to other Figures.

The analyser 100 is shown having one or more buttons or similar elements 102 for operating the analyser by an operator (e.g. doctor, nurse, etc.). The analyser may further comprise e.g. a display (not shown) for displaying operational data, such as e.g. data indicating the results of an analysis carried out by the analyser. The handgrip 103 is shown comprising two flanges 104, 105 for pressing against corresponding end sides of the analyser 100 to keep the analyser press fitted into the handgrip 103. These flanges 104, 105 may be comprised in a casing of the handgrip 103, into which the analyser 100 may be press fitted by the flanges 104, 105. In some embodiments, more than two flanges differently arranged (e.g. for pressing against respective lateral sides of the analyser 100) may be used to keep the analyser 100 press fitted into the handgrip 103 (or casing of the handgrip 103).

Figure 2 schematically represents a lateral view of an analyser 100 press fitted into a handgrip 103 similar to the one shown in Figure 1. In this case, further elements with respect to Figure 1 are shown. In particular, a first sheet-shaped portion 200 (which may be part of a casing of the handgrip 103) is shown connecting the flanges 104, 105, and a second sheet-shaped portion 201 (which may be part of the holding device) is shown slidably coupled with the first sheet-shaped portion 200. Details about this slidably coupling are provided in other parts of the description with reference to other Figures. In other embodiments, both sheet-shaped portions 200, 201 may be shaped and arranged relative to each other in such a way that the portion 200 would not be visible in a lateral view similar to the one depicted by Figure 2.

The first sheet-shaped portion 200 may (completely or partially) cover a rear/bottom side of the analyser 100. The second sheet-shaped portion 201 is shown having an external rough surface which may be based on a plurality of small protuberances 202. This rough surface 202 may provide an improved contact by an operator's hand (e.g. fingers) on the second sheet-shaped portion 201. This improved contact may facilitate the operator to cause the second sheet-shaped portion 201 to slide with respect to the first sheet-shaped portion 200.

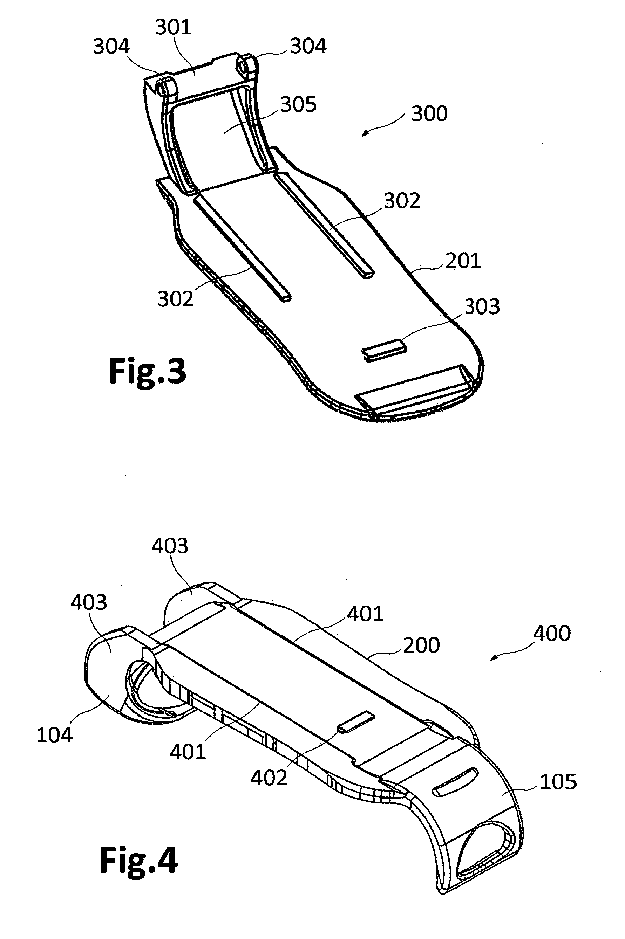

Figure 3 schematically represents a perspective view of a holding device portion 300, and Figure 4 schematically represents a perspective view of a casing 400. Both the holding device portion 300 and the casing 400 are suitable for a handgrip 103 similar to the ones shown in previous Figures. The holding device portion 300 is shown with a sheet-shaped portion 201 having corresponding rails 302 arranged to be guided by suitable guiding elements 401 comprised in a sheet-shaped portion 200 of the casing 400 (compatible with the holding device 300). This rail based configuration 302, 401 permits the sheet-shaped portion 201 of the holding device portion 300 (i.e. of the holding device) to slidably move with respect to the sheet-shaped portion 200 of the casing 400.

The sheet-shaped portion 201 of the holding device 300 may also comprise a stopping element 303 arranged to be stopped (during the abovementioned sliding movement) by a stopper 402 comprised in the sheet-shaped portion 200 of the casing 400. This stopping configuration 303, 402 may permit limiting to some extend the sliding motion of the sheet-shaped portion 201 of the holding device portion 300 (i.e. of the holding device) with respect to the sheet-shaped portion 200 of the casing 400. In other embodiments, a configuration based on suitably formed grooves may be used for implementing the same or similar limitation of the sliding motion of the sheet-shaped portion 201 (of the holding device 300) with respect to the sheet-shaped portion 200 (of the casing 400).

The holding device portion 300 is shown further comprising a substantially upwards extension 305 with respect to the sheet-shaped portion 201. An end of this upwards extension 305 may constitute a lower pressing member 301 of a pressing arrangement of the holding device. This upwards extension 305 may comprise corresponding "pivot" pieces 304 for pivotally coupling an upper pressing member (not shown) with the lower pressing member 301. These "pivot" pieces 304 may comprise corresponding holes for passing a pin through them to form said pivotally coupling of the upper pressing member with respect to the lower pressing member 301.

The casing 400 is shown comprising two flanges 104, 105 for pressing against corresponding end sides of an analyser to keep the analyser press fitted into the casing 400. A flange 104 focused to press against the side of the analyser having a groove (into which an analysis strip can be inserted) may have a configuration suitable for "laterally" hiding/protecting the abovementioned upwards extension 305 (and upper pressing member) of the holding device 300. In particular, the flange 104 is shown in Figure 4 comprising two walls 403 arranged in such a way that the upwards extension 305 (and upper pressing member) is laterally protected by said walls 403, when the holding device 300 is in the proximal (or receded) position (with respect to the groove).

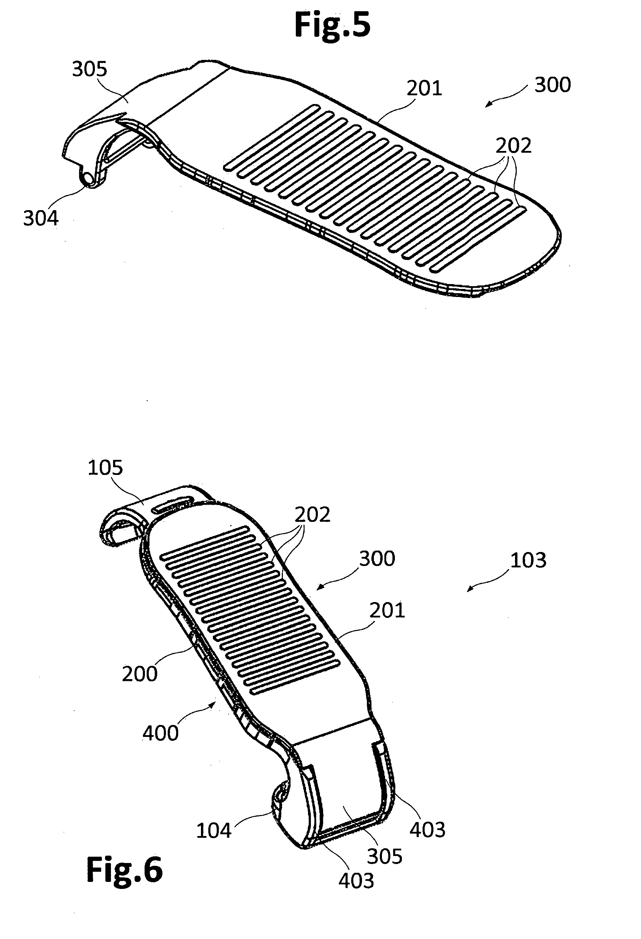

Figure 5 schematically represents a perspective view of a holding device portion 300 similar to the one shown in Figure 3, but turned upside down with respect to the position shown in Figure 3. In Figure 5, the sheet-shaped portion 201 of the holding device 300 is shown having a rough external surface, which is based on having a plurality of slightly protruding rows 202.

Figure 6 schematically represents a perspective view of a handgrip 103 similar to the ones shown in previous Figures. In particular, this handgrip 103 is formed by a handgrip casing 400 similar to the one shown in Figure 4, and a holding device 300 similar to the one shown in Figures 3 and 5. The handgrip 103 is shown with the holding device 300 in the proximal (or receded) position, in which the upwards extension 305 (and upper pressing member) of the holding device 300 is laterally covered by corresponding walls 403 of the flange 104 of the casing 400. This lateral covering by the walls 403 may be seen as constituting a guide or receptacle for housing the upwards extension 305 (and upper pressing member) when the holding device 300 in the proximal (or receded) position.

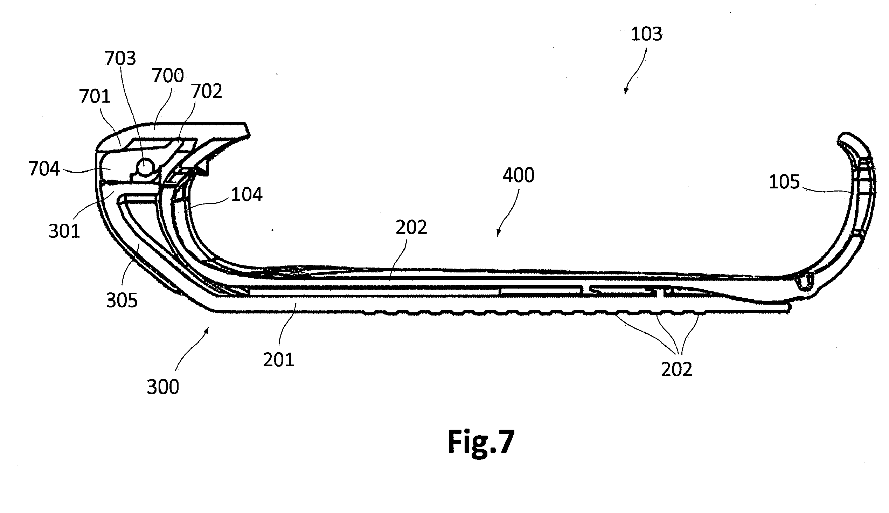

Figure 7 schematically represents a sectional view of a handgrip 103 similar to the ones shown in previous Figures, said sectional view having been taken according to a vertical longitudinal plane 106 of the handgrip 103 (see dashed line of Figure 1). The handgrip 103 of Figure 7 is also formed by a handgrip casing 400 similar to the one shown in Figure 4, and a holding device 300 similar to the one shown in Figures 3 and 5. In this case, a complete pressing arrangement is shown comprising an upper pressing member 704 and a lower pressing member 301. The upper pressing member 704 is shown pivotally coupled to the lower pressing member 301 by a suitable pin 703, in such a way that only the upper pressing member 704 may pivot (with respect to the lower pressing member 301). The pin 703 may be a separate piece (coupling the upper pressing member 704 to the lower pressing member 301), even though, in other embodiments, the pin 703 may be a portion of the upper pressing member 704 itself.

In alternative implementations, the lower pressing member 301 may be pivotally coupled to the upper pressing member 704, in such a way that only the lower pressing member 301 may pivot (with respect to the upper pressing member 704). In other alternative configurations, both the upper and lower pressing members 301, 704 may be coupled pivotally to each other, such that both pressing members 301, 704 may pivot (relative to each other).

The flange 104 is shown comprising a top wall 700 above covering the pressing arrangement 301, 704. This top wall 700 is shown comprising (at its outer end) a substantially downwards extension 701, and the upper pressing member 704 is shown comprising (at its inner end) a substantially upwards extension 702. These extensions 701, 702 are shaped and arranged relative to each other in such a way that, depending on whether they engage (in contact) or not, the upper pressing member 704 makes a pressure on a possibly inserted strip(and, optionally, on the lower pressing member 301) or ceases said pressure. Details about these two situations (making or ceasing the pressure) of the pressing arrangement are provided in other parts of the description with reference to other Figures.

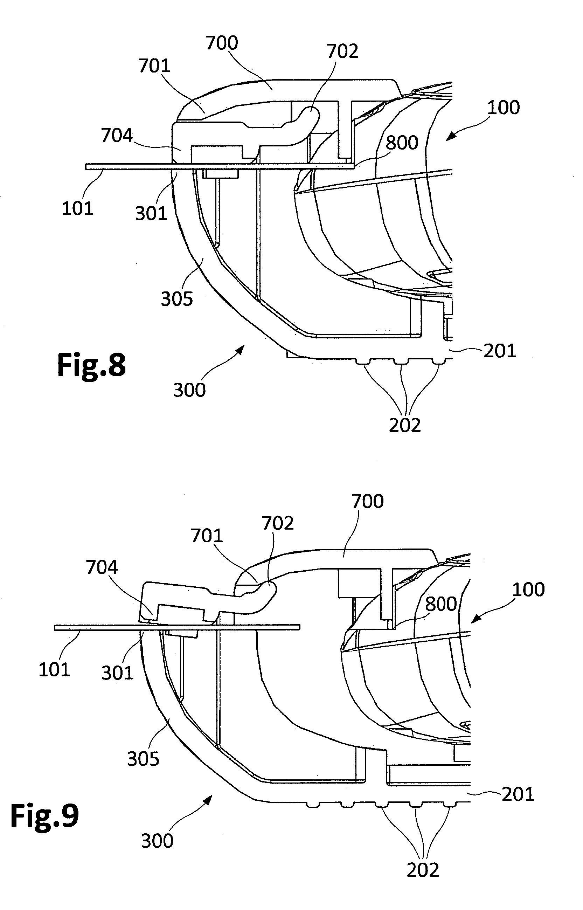

Figure 8 schematically represents a sectional view of a portion of a handgrip 103 similar to the ones shown in previous Figures, with an analyser 100 press fitted into the handgrip 103 and a strip 101 inserted into a pertinent groove 800 of the analyser 100.The sectional view has been taken according to a vertical longitudinal plane 106 of the handgrip 103(see dashed line of Figure 1). In this case, the pressing arrangement 301, 704 (of the holding device) is shown in the proximal (or receded) position (relative to the groove). Note that the upper upwards extension 702 of the upper pressing member 704 is not in contact (engaged) with the downwards extension 701 of the top wall 700, which causes the upper pressing member 704 to be making a pressure on the strip 101 (and the lower pressing member 301).

Once the strip 101 has been used by the analyser 100 (e.g. a desired analysis has been completed), an operator (e.g. doctor, nurse, etc.) may act on the sheet-shaped portion 201 of the holding device for causing it to slide with respect to the casing 400 of the handgrip 103. This sliding movement may cause the pressing arrangement 301, 704 to move from the proximal (or receded) position (with respect to the groove 800) to a position in which the pressing arrangement 301, 704 is still making pressure on the strip 101, so the strip 101 is pulled and finally extracted from the groove 800. After this, the sliding movement caused by the user may be continued until a new position is achieved.

In this new position, the upwards extension 702 of the upper pressing member 704 comes into contact (engages) with the downwards extension 701 of the top wall 700. From this moment, the upper pressing member 704 starts to pivot with respect to the lower pressing member 301, in such a way that the upper pressing member 704 starts to cease the pressure on the strip 101 (and the lower pressing member 301). The upper pressing member 704 pivots until the distal (or extended) position (of the pressing arrangement 301, 704 relative to the groove 800) is achieved, in which case the pressure on the strip 101 is completely ceased, such that the strip 101 is released.

Figure 9 schematically represents a sectional view of the handgrip 103 portion of Figure 8, with the pressing arrangement 301, 704 in the distal (or extended) position, i.e. the strip 101 being finally ejected. This sectional view has been taken according to a vertical longitudinal plane 106 of the handgrip 103(see dashed line of Figure 1). The holding device (i.e. the pressing arrangement 301, 704) is shown after having pulled the strip 101 and, so, completely removed the strip 101 from the groove 800. As the strip 101 is no longer stuck between the upper pressing member 704 and the lower pressing member 301, the strip 101 may be thrown away by e.g. suitably tilting the handgrip 103 and analyser 100 (which is press fitted into the handgrip 103).

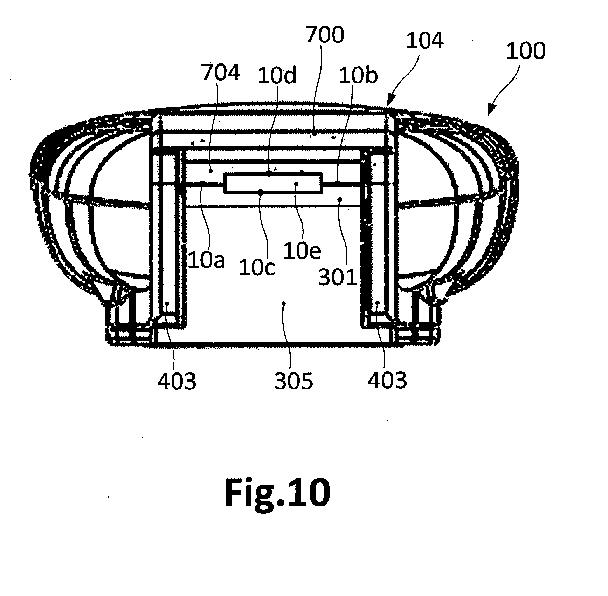

Figure 10 schematically represents a view of an analyser 100 press fitted into a handgrip 103 similar to the ones shown in previous Figures, said view being taken from a particular point of view 203 indicated in Figure 2. Figure 10 shows a pressing arrangement constituted by an upper pressing member 704 and a lower pressing member 301 in the proximal position. In this position, the pressing arrangement 301, 704 is above covered by a top wall 700 of a corresponding flange 104 and laterally protected by lateral walls 403 of the flange 104.

The upper pressing member 704 is shown having a recess 10d and the lower pressing member 301 is shown also having a recess 10c. Both recesses 10c, 10d are configured to be facing in alignment respective to each other, when the pressing member is in the proximal position, such that a kind of tunnel-shaped cavity 10e is formed. This tunnel-shaped cavity 10e connects a groove of the analyser 100 into which an analysis strip is to be inserted and the outside, such that the strip is inserted into the groove by first passing through this tunnel-shaped cavity 10e.

The tunnel-shaped cavity 10e (i.e. the recesses 10c, 10d) is shaped/sized in such a manner that the strip passes under a certain pressure through the tunnel-shaped cavity 10e. This pressure may be, in turn, enough to pull the strip from the groove when the pressing arrangement 301, 704 is moved from the proximal (or receded) position to the distal (or extended) position. Some or all of the internal surfaces of the tunnel-shaped cavity 10e may be rough surfaces, such that pulling of the strip by the pressing arrangement may be improved. In other embodiments, none of said internal surfaces are rough surfaces.

In alternative embodiments, only the upper pressing member 704 may have a recess 10d, or only the lower pressing member 301 may have a recess 10c, or neither may have a recess. In this last case, the upper pressing member 704 may be pivotally coupled to the lower pressing member 301 in such a manner that, in the proximal (or receded) position, there may be a suitable distance between them, for permitting both insertion (under pressure) of the strip and pulling (by the pressing arrangement) of the strip.

An aspect of having the recesses 10c, 10d shown in Figure 10 may be that the height of the tunnel-shaped cavity 10e may be better ensured and remained invariable (along the life of the handgrip) whenever the pressing arrangement is in the proximal position. This results in that the pressure on the strip may be kept optimally invariable (along the life of the handgrip) whenever the pressing arrangement is in the proximal position. A key point for achieving this property may be that the upper and lower pressing members have respective contacting surfaces 10a, 10b at both sides of the recesses 10c, 10d, which causes a significantly stable contact between the upper and lower pressing members(at both sides of the tunnel-shaped cavity 10e).

In some or all of the described embodiments, the handgrip may be manufactured either separated from the analyser or integrated with the analyser. In the former case, the handgrip may be a removable handgrip, such that it may be provided individually. On the contrary, in the latter case, the analyser may be provided with the handgrip as a single piece. An aspect of providing a removable handgrip may be that a damaged handgrip may be easily replaced by another in a very easy and cheap manner. Another aspect of providing a removable handgrip may be that a same handgrip may be reused, applied to different analysers of the same type/shape/size. Of course, different sizes/shapes for the removable handgrip may be taken into account depending on the model of analyser with which the handgrip is intended to be used.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described. Thus, the scope of the present invention should not be limited by particular embodiments, but should be determined only by a fair reading of the claims that follow.

## Claims

1. A handgrip for an analyser, the analyser having a groove into which an analysis strip can be inserted, the handgrip comprising a movable holding device that can be moved between a proximal position and a distal position relative to the groove, wherein the holding device is adapted to retain the analysis strip when the holding device is in the proximal position and to release the analysis strip when the holding device is in the distal position.

2. A handgrip according to claim 1, wherein the holding device comprises a pressing arrangement adapted to retain the analysis strip by applying a pressure when the holding device is in the proximal position and to release the analysis strip by ceasing the pressure when the holding device is in the distal position.

3. A handgrip according to claim 2, wherein the pressing arrangement comprises an upper pressing member and a lower pressing member adapted to apply the pressure to the analysis strip and to cause the analysis strip to be stuck between the upper and lower pressing members.

4. A handgrip according to claim 3, wherein at least one of the upper and lower pressing members has a recess such that the analysis strip is substantially press fitted into the recess when the analysis strip is stuck between the upper and lower pressing members.

5. A handgrip according to claim 4, wherein each of the upper and lower pressing members has a recess such that the analysis strip is substantially press fitted into both recesses when the analysis strip is stuck between the upper and lower pressing members.

6. A handgrip according to any of claims 3 to 5, wherein the upper and lower pressing members are coupled pivotally to each other, such that the upper and lower pressing members can pivot relative to each other for causing the analysis strip to swap between being stuck and not being stuck between the upper and lower pressing members, and vice versa.

7. A handgrip according to any of claims 3 to 5, wherein the upper pressing member is coupled pivotally to the lower pressing member, such that the upper pressing member can pivot relative to the lower pressing member for causing the analysis strip to swap between being stuck and not being stuck between the upper and lower pressing members, and vice versa.

8. A handgrip according to claim 7, wherein the upper pressing member comprises an upwards extension at its inner end.

9. A handgrip according to any of claims 1 to 8, comprising a casing configured to receive the analyser in a press fitting manner; wherein the holding device is movable relative to the casing.

10. A handgrip according to claim 9, wherein the casing comprises at least two flanges for pressing against corresponding end sides of the analyser to keep the analyser press fitted into the casing.

11. A handgrip according to claim 10, wherein one of the flanges is adapted to press against the end side of the analyser substantially having the groove; wherein this flange comprises a top wall having a downwards extension at its outer end; wherein the downwards extension of the top wall and the upwards extension of the upper pressing member are arranged respective to each other in such a way that they engage during a part of the motion of the holding device between the proximal and distal positions; wherein said engagement causes the pivoting of the upper pressing member with respect to the lower pressing member.

12. A handgrip according to any of claims 10 or 11, wherein the casing comprises a sheet-shaped portion connecting the at least two flanges, in such a way that a rear/bottom side of the analyser is at least partially covered by the sheet-shaped portion when the analyser is press-fitted into the casing.

13. A handgrip according to claim 12, wherein the holding device comprises a sheet-shaped portion slidably coupled, in particular by a rail based mechanism, to the sheet-shaped portion of the casing.

14. A handgrip according to claim 13, wherein the sheet-shaped portion of the holding device has an external rough surface.

15. A handgrip according to any of claims 1 to 14, wherein the handgrip is a removable handgrip, such that the handgrip may be easily removed from the analyser.
